# EUROPEAN PATENT SPECIFICATION

(11) **EP 2 794 423 B1**
(45) Date of publication and mention of the grant of the patent: **28.09.2016**
(21) Application number: 12816176.7
(22) Date of filing: 11.12.2012
(51) Int. Cl.: B65D 75/58, A61F 13/551

(54) **PACKAGE COMPRISING PEELABLE OVERLABEL**
VERPACKUNG MIT ABLÖSBAREM ÜBERETIKETT
PAQUET COMPRENANT UNE SURÉTIQUETTE PELABLE

(30) Priority: 21.12.2011 US 201161578623 P
(43) Date of publication of application: 29.10.2014
(73) Proprietor: The Procter & Gamble Company, Cincinnati, OH 45202 (US)
(72) Inventor: SCHICKLI, Eric, Mitchell, Cincinnati, Ohio 45224 (US); SOUZA, David, Christopher, San Rafael, California 94903 (US); WASSON, Matthew, Howard, Cincinnati, Ohio 45217 (US)
(74) Representative: Mather, Peter Geoffrey
(86) International application number: PCT/US2012/068942
(87) International publication number: WO 2013/096007

(56) References cited:
- WO-A1-2011/012649
- GB-A- 2 307 673
- US-A1- 2008 032 081

## Description

### FIELD OF THE INVENTION

The present invention relates to packages according to the preamble of claim 1, which are known e.g. from WO 2011/012 649.

### BACKGROUND OF THE INVENTION

Packages containing an access opening that is covered by a resealable overlabel are commonly used for storing consumer goods wherein less than the entire purchased product is used at one particular point in time. For example, wet wipes can be packaged in a flexible film package that employs a resealable overlabel. A user removes one or more wipes from the package and then reseals it to preserve the cleanliness and moisture level of the unused wipes for future use.

The overlabel on many of these resealable packages is intended to stay connected to the package and simply be peeled back sufficiently to provide access to the package's contents. For example, the overlabel may be permanently affixed on one of its edges and releasably affixed on the remaining edges. Pressure sensitive adhesive is generally used to releasably affix portions of the overlabel to the underlying package substrate. The adhesive must possess sufficiently high strength to maintain adhesion of the overlabel to the package substrate prior to use by a consumer, but not too much strength (or tack) wherein the initial peeling force can lead to tearing, delaminating, or distorting the package and/or can result in a consumer peeling the label completely off of the underlying substrate. The inventors of the present invention have discovered that simply optimizing adhesive strength can be insufficient to deliver the right force balance for the package and the consumer using the same.

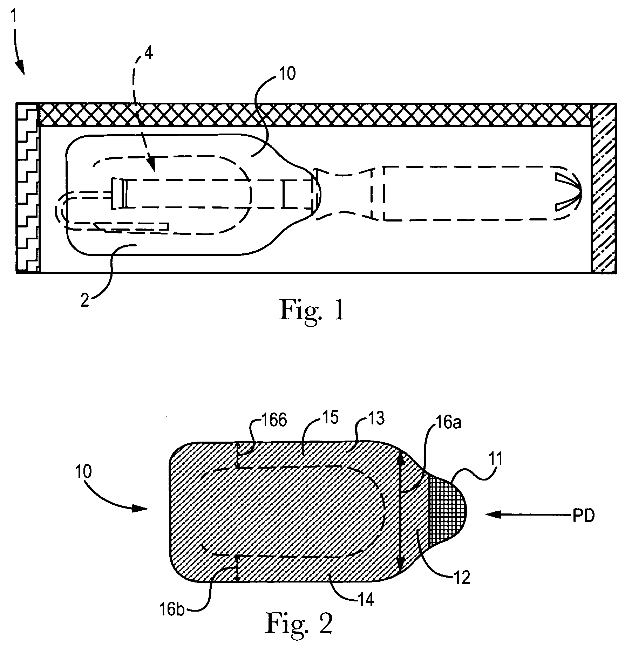

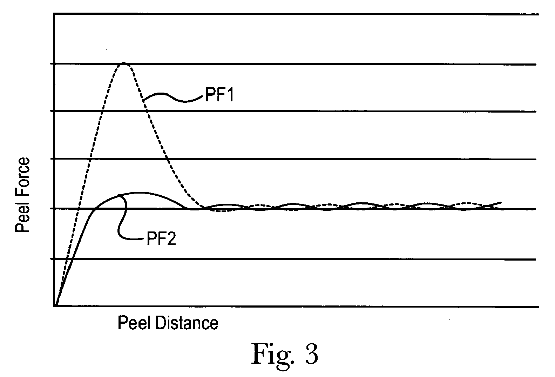

One of the factors that leads to a high initial peel force is that the adhesive wet edge width of an overlabel that seals around the perimeter of an opening can vary greatly, with a larger wet edge width needing to be overcome at the beginning stages of peeling the overlabel. A high initial peel force diminishes after the first portions of the overlabel are released from the substrate. This initial high peel force can compromise the integrity of the package for less than optimal continued use. The initial high peel force can also cause a consumer to accelerate the peeling action after the initial peel force is overcome that can lead to the overlabel being torn partially or completely away from the package substrate. Figures 1 and 2, respectively, show an exemplary package 1 that includes an overlabel 10 positioned over a package opening 4. Overlabel 10 has a grasping tab 11, and three edges (12, 13, and 14) that each contain an adhesive 15 for releasable attachment to the package substrate portions surrounding opening 4. Note that the substrate inside of opening 4 is diecut and therefore the label and underlying substrate can be lifted together essentially without any additional resistance. When a consumer grasps tab 11 and begins to peel overlabel 10 from substrate 2 (PD= peel direction), edge 12 is the first edge to be released from the substrate surrounding package opening 4. However, as can be seen in Figure 2, the adhesive wet edge width 16a is significantly greater on edge 12 than the wet edge width 16b on each of overlabel edges 13 and 14. This can result in a peel force profile PF1 like that shown in Figure 3. Again, this initial spike in peel force may compromise the integrity of the underlying package substrate or result in the overlabel thereafter being peeled too far.

The present invention addresses one or more of the above-described issues.

### BRIEF DESCRIPTION OF THE DRAWINGS

The following detailed description of specific embodiments of the present invention can be best understood when read in conjunction with the drawings enclosed herewith.
Figure 1 is plan view of a package that includes an overlabel positioned over a package opening.
Figure 2 is plan view of a package overlabel.
Figure 3 is a peel force chart.
Figure 4 is a plan view of a package that includes an overlabel positioned over a package opening.
Figure 5 is a plan view of a first embodiment of an overlabel comprising adhesive and adhesive deadener.
Figure 6 is a plan view of a second embodiment of an overlabel comprising adhesive and adhesive deadener.
Figure 7 is a plan view of a third embodiment of an overlabel comprising adhesive and adhesive deadener.
Figure 8 is a plan view of a fourth embodiment of an overlabel comprising adhesive and adhesive deadener.

### DETAILED DESCRIPTION OF THE INVENTION

The following text sets forth a broad description of numerous different embodiments of the present invention. The description is to be construed as exemplary only and does not describe every possible embodiment since describing every possible embodiment would be impractical, if not impossible. Numerous alternative embodiments could be implemented, using either current technology or technology developed after the filing date of this patent, which could still fall within the scope of the claims.

It should also be understood that, unless a term is expressly defined in this specification using the sentence "As used herein, the term '_' is hereby defined to mean..." or a similar sentence, there is no intent to limit the meaning of that term, either expressly or by implication, beyond its plain or ordinary meaning, and such term should not be interpreted to be limited in scope based on any statement made in any section of this patent (other than the language of the claims). No term is intended to be essential to the present invention unless so stated. To the extent that any term recited in the claims at the end of this patent is referred to in this patent in a manner consistent with a single meaning, that is done for sake of clarity only so as to not confuse the reader, and it is not intended that such a claim term be limited, by implication or otherwise, to that single meaning.

Embodiments described herein relate to packages containing an applicator tampon. The consumer goods may be disposable, durable or semi-durable. The packages provided herein comprise an opening that is covered by an overlabel. The overlabel is at least partially peeled off of the package so that articles contained within the package can be removed through the now exposed opening. In some embodiments, the package can be reclosed by covering the opening with the overlabel. A consumer may wish to dispose of a used article by placing the article back into the package via the opening and re-adhering the overlabel. Used applicator tampons that are soiled may benefit from a package embodiment that includes an absorbent interior layer (e.g., a nonwoven layer). Packages of the present invention may be constructed from a substrate that has a single material layer or a laminate substrate that has two or more similar or dissimilar material layers. For example, one package embodiment is made from a polymeric film - nonwoven laminate wherein the film forms the exterior surface of the package and the nonwoven forms the interior surface of the package.

As used herein, the term "absorbent" refers to materials that can hold, retain, entrap, and/or contain body fluids.

As used herein, the term "absorbent article" refers to an applicator tampon.

The term "disposable" is used herein to describe articles that are intended to be discarded after a single use. That is, they are not intended to be laundered or otherwise restored or reused.

As used herein, the term "feminine hygiene articles" refers to an applicator tampon.

As used herein, the term "nonwoven" can refer to a web or fabric having a structure of individual fibers or threads which are interlaid, but not in a regular, repeating manner as in a woven or knitted fabric. Nonwoven webs or fabrics can be formed from many processes, such as, for example, meltblowing processes, spunbonding processes, hydroentangling processes, and bonded carded web processes.

As used herein, the term "tampon" refers to any type of absorbent structure such as, *e.g*., an absorbent mass, that can be inserted into the vaginal canal or other body cavity for the purpose of, such as, *e.g.,* absorbing fluid, aiding in wound healing, and/or for delivering materials, such as moisture or active materials such as medicaments. The term "tampon" includes the combination of an absorbent structure with any type of applicator that can be associated with the absorbent structure to facilitate insertion of a tampon into the vaginal canal or other body cavity. A tampon can include any known tampon configuration such as, for example, digital tampons, tampons with traditional plunger type applicators, and/or tampons with compact applicators, such as, *e.g.,* tampons described in U.S. Patent Nos. 4,726,805; 4,846,802; 4,960,417; 5,087,239; 5,279,541; 6,258,075; 6,478,763; or any other tampon.

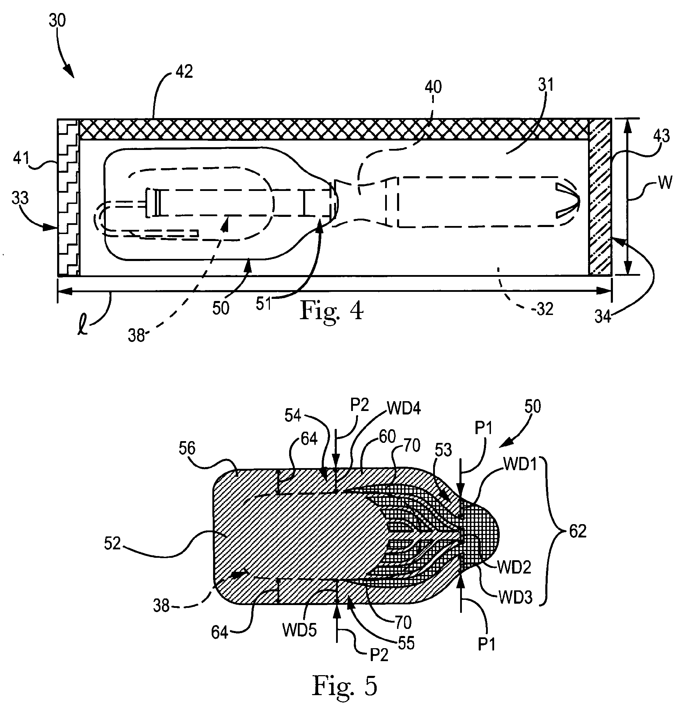

Figure 4 illustrates an exemplary package 30 of the present invention. Package 30 has an outer surface 31, an inner surface 32, a top 33, a bottom 34, a length (1), and a width (w). As shown in Figure 4, package 30 also has a first side and a second side, and at least one of the sides can include fold. Package 30 can substantially enclose a consumer good such as, for example, a tampon applicator 40. As shown in Figure 4, the package includes an opening 38 covered by an exemplary overlabel 50. The opening can be provided in any suitable shape, such as, for example, a circle, a crescent, an oval, a semicircle, a horseshoe, an ellipse, a hexagon, an octagon, a pentagon, a star, a triangle, a rectangle, an irregular shape, a symmetrical shape, a non-symmetrical shape, or any other suitable shape. The opening can be provided with any suitable method, such as, for example, cutting, such as, die cutting or laser cutting, scoring, such as laser scoring, or perforating, such as, mechanical perforations, or any other suitable method. In addition, the package is shown having three side seals, 41, 42, and 43. In certain embodiments, overlabel 50 can have a grasping tab 51.

Overlabel 50 is shown and described in greater detail with reference to Figure 5. Overlabel 50 has opposing first and second transverse edges 52 and 53, and opposing third and fourth longitudinal edges 54 and 55. It should be understood that the geometry of the overlabel can vary greatly from that of exemplary overlabel 50. And that the interface of the opposing first and second edges with the opposing third and fourth edges may not be as distinct as those associated with overlabel 50; that is, the overlabel may be circular such that the recited first through fourth edges are defined by different portions of the perimeter of the circle. Any suitable overlabel for sealing the package can be used, such as overlabels formed from materials such as, e.g., polypropylene, polyesters, acetate, vinyl, polyethylene terephthalate, foil, wax, resin, paper, nonwoven, or any other suitable material. The overlabel can be attached to the package substrate in any suitable manner, such as, for example, using pressure sensitive adhesives, heat activated adhesives, hot melt adhesives, solvent based adhesives, water based adhesives, glue, or any other suitable adhesive. The overlabel can be any suitable thickness, such as, for example, from 1.5 to 5.0 mils in thickness.

The substrate-facing surface 56 of overlabel 50 is shown in Figure 5 and a broken reference line is included to illustrate the corresponding package substrate opening 38. Adhesive 60 is disposed on the transverse and longitudinal edges, and is shown via diagonal cross-hatching. Adhesive deadener 70 (illustrated with dual-direction cross-hatching) is applied over portions of adhesive 60 that is located on transverse edge 53 so that the adhesive wet edge width 62 associated with edge 53 is closer in dimension to the adhesive wet edge width 64 that is associated with each of longitudinal edges 54 and 55. Norminalizing the adhesive wet edge width that needs to be overcome during the peeling process will result in a more uniform peel force profile, which in turn, can minimize damage to the underlying package substrate and/or reduce the likelihood of a consumer peeling the overlabel further than it was designed to be.

A variety of adhesives can be used on the overlabels of the present invention. These can include, for example, acrylic emulsions, solvent based adhesives, and hot melts. One preferred class of adhesives is pressure sensitive adhesives. Exemplary hot melt adhesives include styrenic block copolymers adhesives. Adhesives can be applied by any known technique, including via slot coating and printing (e.g., flexography). As used herein, "adhesive deadener" means any substance that can be brought into contact with adhesive to reduce the level of tack of the adhesive, including deadeners, detackifiers (e.g., talc), and ink/pigments. Adhesive deadeners can include those known in the art of packaging and labels. An exemplary adhesive deadener useful for the present invention is a UV-cured varnish. Adhesive deadeners can be applied by any know method, including, for example, via flexography.

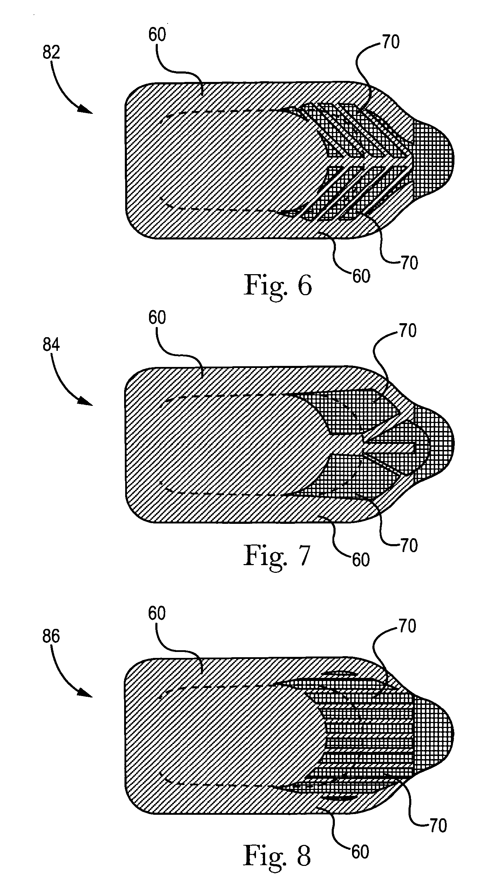

Figures 6-8 include other overlabel embodiments (82, 84, and 86, respectively) that have varying patterns of adhesive deadener 70 applied to portions of the previously applied adhesive 60. It should be understood that while adhesive deadener is applied most significantly to adhesive on the second edge of the overlabels shown in Figures 4-6, adhesive deadener in similar or varying amounts can be applied to adhesive on more than one edge of the overlabel.

Application of deadener on the adhesive located on overlabel edge 53 results in a discontinuous pattern of adhesive. And since longitudinal edges 54 and 55 have portions along their lengths that do not contain any deadener in the embodiments shown in Figures 5-8, these edges comprise a continuous pattern of adhesive. In alternative embodiments, adhesive is applied to edges of the overlabel in a pattern such that a subsequent application of a deadener is not required. Thus, a designed pattern of active adhesive can be achieved in two ways: 1) applying a first layer of adhesive and then applying a second partial layer of adhesive deadener; and 2) applying a layer of adhesive in a discontinuous pattern to manage the effective adhesive wet edge width. As used herein, the term "active adhesive" refers to the adhesive zone that comes into contact with the package substrate surrounding the opening and that is used reseal the overlabel to the package substrate (for example, the adhesive zone that surrounds the opening up to the terminal end of the broken line proximate the transverse edge 52 in Figure 5). The package opening can be partially or completely die-cut such that a flap of package substrate becomes substantially permanently attached to a central portion of the overlabel. Thus, adhesive on this central portion of the overlabel is initially active, but does not continue to be active after it contacts a piece of die-cut package substrate. Then, only the adhesive that comes into contact with the package substrate along the outside perimeter of the opening is "active adhesive" to permit the overlabel to be peeled back to expose the opening and then refastened (at least once) to the package to cover up the opening. As used herein, the term "discontinuous" means a purposeful break in adhesive application and does not mean a discontinuity resulting from an inherent adhesive application process such as, for example, a swirled adhesive application.

Preferably, a wet edge width dimension of active adhesive that contacts the package substrate around the outer perimeter of the package opening is substantially the same along the length of the overlabel. In some embodiments, "substantially the same" is met where a minimum of the width dimension of active adhesive is no less than 70%, 80% or 90% of a maximum width dimension of active adhesive along the length of the overlabel from a starting edge that is lifted from the package substrate to a final edge that is lifted from the package substrate during the peeling process. By way of example and with reference to Figure 5, exemplary overlabel 50 at a first position P1 proximate transverse edge 53 has a width dimension of active adhesive that is defined by adding WD1, WD2, and WD3 together. At a second position P2 along the length of overlabel 50, the width dimension of active adhesive is defined by adding WD4 and WD5 together. If the combination of WD4 and WD5 represents a minimum wet edge width and the combination of W1, W2, and W3 represent a maximum wet edge width, then the inventors consider the width dimension of active adhesive that contacts the package substrate around the perimeter of the opening to be substantially the same along the overlabel length where the minimum is no less than 70% of the maximum. The minimum of the width dimension of active adhesive can however be 30-70% of the maximum width dimension of active adhesive. In other embodiments, "substantially the same" is met where an average width dimension of active adhesive is no less than 60%, 70%, 80% or 90% of a maximum width dimension of active adhesive along the length of the overlabel from a starting edge that is lifted from the package substrate to a final edge that is lifted from the package substrate during the peeling process.

As discussed above, the present invention generally relates to methods of selectively applying adhesive to a package overlabel and/or selectively deadening previously applied adhesive to impart a more uniform peel force required to lift the overlabel off of the underlying package substrate. For example, a desired peel force profile can take the shape profile PF2 that is shown in Figure 3. Peel force measurements can be made on a universal constant rate of elongation tensile tester with computer interface. The rate used during testing is 100 inches per minute (2.54 meters per minute). Average and peak peel force measurements can be made to determine how uniform the peel force is.

Packages according to the present invention can be formed of any suitable substrate material, such as, for example, a polymeric film comprising polyolefins, polyesters, polyamides, polyvinyl chlorides, ethylene-vinyl acetate copolymers, and/or other suitable films, a nonwoven, a formed film, a paper, or a fabric comprised of suitable material such as polyethylene, polypropylene, polyester, cellulose, rayon, cotton, super absorbent material such as polyacrylate, or combinations thereof. The package substrate material can be any suitable thickness, such as, for example, greater than about 0.1 mm thick, such as, e.g., greater than about 0.2 mm thick, greater than about 0.3 mm thick, greater than about 0.4 mm thick, greater than about 0.5 mm thick, greater than about 0.6 mm thick, greater than about 0.7 mm thick, greater than about 0.8 mm thick, greater than about 0.8 mm thick, greater than about 0.9 mm thick, greater than about 1 mm thick, greater than about 2 mm thick, greater than about 3 mm thick, greater than about 4 mm thick, greater than about 5 mm thick, or any other suitable thickness. In addition, in certain embodiments, the package substrate material can have a high tear resistance. In certain embodiments, the package substrate material can be considered non-flushable, non-water-degradable, and/or generally insoluble in water.

The package substrate may comprise a single layer or component. In certain embodiments, the package substrate material can be a multiple component material that can have a first component and a second component. For example, the first component of the package material can be a polymeric film and the second component can be an absorbent material, such as, e.g., a nonwoven, a formed film, a paper, or a fabric. The first component and the second component can be joined in any suitable manner to form the package substrate material, such as, e.g., by adhesive bonding, mechanical bonding, thermal bonding, ultrasonic bonding, extrusion lamination, and the like. While complete bonding of the first component and the second component may not be necessary, in certain embodiments, the bonding should be sufficient to facilitate that the components act as a unit, e.g., bending out-of-plane together.

The layers/components can have a thickness of from about .0005" (~.01mm) to .003" (.07mm). In certain embodiments, the substrate materials can have a basis weight of less than about 50 gsm, such as, e.g., less than about 40 gsm, less than about 30 gsm, less than about 25 gsm, less than about 20 gsm, less than about 15 gsm, or less than about 10 gsm, or any other suitable basis weight. The package substrate materials can be printable, such as, e.g., printed with one or more images, such as, e.g., printed with one or more product features, benefits, or selection guides.

In certain embodiments, the package is sealed around the consumer good on three or more sides, such as, for example, with permanent seals. In addition, the package can include a fold or a permanent seal on the fourth side, such that the consumer good is sealed within the package on all sides. The package includes an opening suitable for removal of the consumer good from the package. In certain embodiments, the opening is provided substantially or entirely on a single face of the package, such as, for example, the front face or the back face of the package. In addition, the package has an overlabel substantially covering the opening. In certain embodiments, the overlabel can cover the entire opening. Alternatively, the overlabel can cover a portion of the opening, such as, for example, a first cut through area of the opening. In this instance, as the label is peeled back, the rest of the opening is developed as the user breaks adjoining perforations defining the opening area.

Packages of the present invention are used for containing applicator tampons. The user can place the used applicator back into the package for disposal. The overlabel can be refastened over the package opening once the used article is placed into the package for transportation and/or disposal, such as, *e.g.,* to provide increased discretion and cleanliness during transportation and/or disposal.

The package can be constructed in any suitable manner, such as, *e.g*., constructed of one connected piece of package material or constructed from multiple pieces of material sufficiently joined together such that it substantially acts as one connected piece of package material. In certain embodiments, the package can be formed by closing the package material via heat-sealing onto itself before and/or after wrapping the absorbent article. In addition, or alternatively, the package can be glued, embossed, crimped, sewed, stitched, entangled, mechanically interlocked, cold pressure welded, ultrasonic bonded, and/or otherwise bonded or sealed.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document, the meaning or definition assigned to that term in this document shall govern.

While particular embodiments of the present invention have been illustrated and described, it would be obvious to those skilled in the art that various other changes and modifications can be made without departing from the scope of the claims. It is therefore intended to cover in the appended claims all such changes and modifications that are within the scope of the claims.

## Claims

1. A package (30) comprising:
a. a package interior;
b. a package substrate;
c. an opening (38) defined in the package substrate to provide access to the package interior;
d. a peelable overlabel (50) affixed to the package substrate to selectively cover the opening and expose the opening, the overlabel comprising opposing first and second edges (52, 53) and opposing third and fourth edges (54, 55) that are situated orthogonal to the first and second edges (52, 53); and
e. adhesive (60) disposed at least partially on a surface of the overlabel (50) facing the package substrate;
f. wherein adhesive deadener (70) is applied over portions of the adhesive (60) that is disposed on the second edge (53);
g. wherein the adhesive on each of the third and fourth edges (54, 55) is devoid of adhesive deadener (70);
**characterized in that** the package interior contains an applicator tampon.

2. The package (30) of claim 1, further comprising a tab (51) extending from the second edge (53) to facilitate lifting the overlabel (50) at least partially off of the substrate.

3. The package (30) of claim 2, wherein the tab (51) comprises adhesive (60) and adhesive deadener (70) covering at least some of the adhesive (60).

4. The package (30) of any one of claims 1 to 3, wherein the package substrate is a laminate of a polymeric film layer and a nonwoven layer.

5. The package (30) of claim 4, wherein the polymeric film layer forms an exterior surface of the package and the nonwoven layer forms an interior surface of the package.

6. The package (30) of claim 1, wherein the overlabel (50) comprises a polymeric film.

7. The package (30) of any one of claims 1 to 6, wherein the package interior contains a disposable article, and wherein the opening (38) is sized and configured to allow the disposable article to be removed from the package (30) without tearing the package substrate.

8. The package (30) of any one of claims 1 to 7, wherein the package (30) is reclosable by covering the opening with the overlabel (50) and pressing the overlabel (50) against the package substrate.

## Patentansprüche

1. Verpackung (30), umfassend:
a. einen Verpackungsinnenbereich;
b. ein Verpackungssubstrat;
c. eine Öffnung (38), die in dem Verpackungssubstrat bestimmt ist, um Zugang zu dem Verpackungsinnenbereich zu ermöglichen;
d. ein abziehbares Überetikett (50), das an dem Verpackungssubstrat befestigt ist, um selektiv die Öffnung zu bedecken und die Öffnung freizulegen, wobei das Überetikett einen gegenüberliegenden ersten und zweiten Rand (52, 53) und einen gegenüberliegenden dritten und vierten Rand (54, 55), die senkrecht zu dem ersten und dem zweiten Rand (52, 53) liegen, umfasst; und
e. Klebstoff (60), der wenigstens teilweise auf einer Oberfläche des Überetiketts (50) angeordnet ist, die dem Verpackungssubstrat zugewandt ist;
f. wobei Klebstoffabschwächer (70) über Abschnitte des Klebstoffs (60) aufgebracht ist, der auf dem zweiten Rand (53) angeordnet ist;
g. wobei der Klebstoff auf jedem des dritten und des vierten Rands (54, 55) frei von Klebstoffabschwächer (70) ist;
**dadurch gekennzeichnet, dass** der Verpackungsinnenbereich einen Applikatortampon enthält.

2. Verpackung (30) nach Anspruch 1, ferner umfassend eine Lasche (51), die sich von dem zweiten Rand (53) aus erstreckt, um das wenigstens teilweise Abheben des Überetiketts (50) von dem Substrat zu erleichtern.

3. Verpackung (30) nach Anspruch 2, wobei die Lasche (51) Klebstoff (60) umfasst und Klebstoffabschwächer (70) wenigstens einen Teil des Klebstoffs (60) bedeckt.

4. Verpackung (30) nach einem der Ansprüche 1 bis 3, wobei das Verpak-kungssubstrat ein Laminat aus einer Polymerfolienschicht und einer Vliesschicht ist.

5. Verpackung (30) nach Anspruch 4, wobei die Polymerfolienschicht eine Außenoberfläche der Verpackung bildet und die Vliesschicht eine Innenoberfläche der Verpackung bildet.

6. Verpackung (30) nach Anspruch 1, wobei das Überetikett (50) eine Polymerfolie umfasst.

7. Verpackung (30) nach einem der Ansprüche 1 bis 6, wobei der Verpak-kungsinnenbereich einen Einwegartikel enthält, und wobei die Öffnung (38) so bemessen und konfiguriert ist, dass der Einwegartikel aus der Verpackung (30) entnommen werden kann, ohne das Verpackungssubstrat zu zerreißen.

8. Verpackung (30) nach einem der Ansprüche 1 bis 7, wobei die Verpackung (30) durch Bedecken der Öffnung mit dem Überetikett (50) und Drücken des Überetiketts (50) gegen das Verpackungssubstrat wiederverschließbar ist.

## Revendications

1. Conditionnement (30) comprenant :
a. un intérieur de conditionnement ;
b. un substrat de conditionnement ;
c. une ouverture (38) définie dans le substrat de conditionnement pour fournir un accès à l'intérieur du conditionnement ;
d. une sur-étiquette décollable (50) fixée sur le substrat de conditionnement afin de recouvrir de façon sélective l'ouverture et d'exposer l'ouverture, la sur-étiquette comprenant des premier et deuxième bords opposés (52, 53) et des troisième et quatrième bords opposés (54, 55) qui sont situés de manière orthogonale par rapport aux premier et deuxième bords (52, 53) ; et
e. de l'adhésif (60) disposé au moins partiellement sur une surface de la sur-étiquette (50) faisant face au substrat de conditionnement ;
f. dans lequel un isolant d'adhésif (70) est appliqué sur des portions de l'adhésif (60) qui est disposé sur le deuxième bord (53) ;
g. dans lequel l'adhésif sur chacun des troisième et quatrième bords (54, 55) est dépourvu d'isolant d'adhésif (70) ;
**caractérisé en ce que** l'intérieur de conditionnement comprend un tampon applicateur.

2. Conditionnement (30) selon la revendication 1, comprenant, en outre, une languette (51) s'étendant depuis le deuxième bord (53) afin de faciliter l'élévation de la sur-étiquette (50) au moins partiellement pour la décoller du substrat.

3. Conditionnement (30) selon la revendication 2, dans lequel la languette (51) comprend un adhésif (60) et un isolant d'adhésif (70) recouvrant au moins une partie de l'adhésif (60).

4. Conditionnement (30) selon l'une quelconque des revendications 1 à 3, dans lequel le substrat de conditionnement est un stratifié d'une couche de film polymère et d'une couche de non tissé.

5. Conditionnement (30) selon la revendication 4, dans lequel la couche de film polymère forme une surface extérieure du conditionnement et la couche de non tissé forme une surface intérieure du conditionnement.

6. Conditionnement (30) selon la revendication 1, dans lequel la sur-étiquette (50) comprend un film polymère.

7. Conditionnement (30) selon l'une quelconque des revendications 1 à 6, dans lequel l'intérieur de conditionnement contient un article jetable et dans lequel l'ouverture (38) est dimensionnée et conçue pour permettre à l'article jetable d'être retiré du conditionnement (30) sans déchirer le substrat de conditionnement.

8. Conditionnement (30) selon l'une quelconque des revendications 1 à 7, dans lequel le conditionnement (30) est refermable par recouvrement de l'ouverture avec la sur-étiquette (50) et pression de la sur-étiquette (50) contre le substrat de conditionnement.
